# DEMANDE DE BREVET EUROPEEN

(11) **EP 2 359 760 A1**
(43) Date de publication de la demande: **24.08.2011**
(21) Numéro de dépôt: 11154447.4
(22) Date de dépôt: 15.02.2011
(51) Int. Cl.: A61B 17/80

(54) **Materiel de fixation de deux parties d'un os l'une a l'autre**

(30) Priorité: 15.02.2010 FR 1051055
(71) Demandeur: Implants International Limited, Cleveland Thornaby-on-Tees TS17 9LZ (GB)
(72) Inventeur: Emmanuel, Mohan, Thornaby-on-tees, Cleveland TS17 9LZ (GB)
(74) Mandataire: Jeannet, Olivier

(57) **Abrégé**

Ce matériel (1) comprend :
- une plaque osseuse (2) destinée à être fixée aux deux parties d'os, et
- une tige (3) coudée à sensiblement quatre-vingt-dix degrés, dont une première branche (3a) est destinée à être engagée dans l'une des deux parties d'os.

Selon l'invention,
- ladite tige (3) est une tige intramédullaire dont ladite première branche (3a) est destinée à être engagée dans le canal médullaire de ladite partie d'os, la deuxième branche (3b) de cette tige étant apte, lorsque ladite première branche (3a) est engagée dans le canal médullaire de ladite partie d'os, à être reliée à la plaque (2), et
- le matériel comprend des moyens (5, 21, 33) de liaison de cette deuxième branche (3b) à la plaque (2).

## Description

La présente invention concerne un matériel de fixation de deux parties d'un os l'une à l'autre.

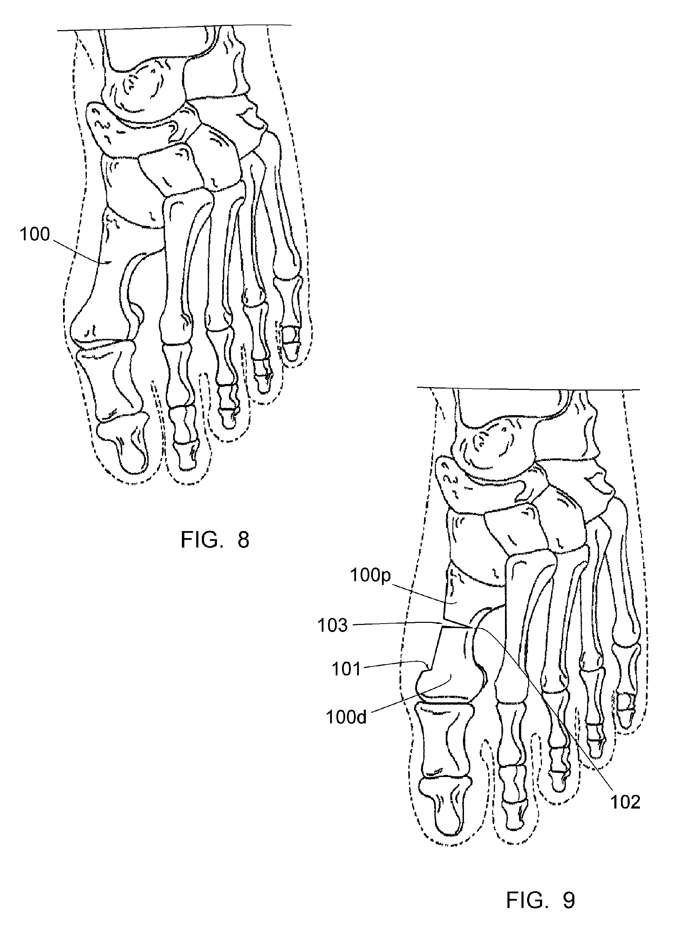

Certains os, en particulier le premier métatarsien, peuvent avoir une position défectueuse, ou peuvent adopter au fil du temps une telle position défectueuse, nécessitant la réalisation d'une chirurgie correctrice de cette position. La figure 8 montre une telle position défectueuse dans le cas d'un premier métatarsien, conduisant à la pathologie connue sous le nom d'hallux valgus.

Un procédé de correction connu consiste, (i) à opérer une découpe transversale dans le premier métatarsien, à partir de la face interne de celui-ci, sur pratiquement la totalité de sa section, c'est-à-dire en laissant subsister une portion réduite de jonction entre les deux parties d'os individualisées par la découpe, propre à permettre un pivotement de la partie distale de l'os par rapport à la partie proximale de celui-ci, (ii) à opérer un pivotement de la partie distale par rapport à la partie proximale dans le sens de l'éloignement des interfaces osseuses de ces parties d'os, puis (iii) à fixer cette partie distale par rapport à cette partie proximale dans cette position pivotée.

Si, selon la position défectueuse du premier métatarsien, une correction doit être opérée dans le sens opposé à celui mentionné ci-dessus, le procédé comprend l'étape (i) ci-dessus, puis (ii) une étape consistant à opérer une coupe osseuse oblique au niveau de chaque interface osseuse, afin d'aménager un espace vide entre ces interfaces, puis (iii) une étape consistant à opérer un pivotement de la partie distale par rapport à la partie proximale dans le sens du rapprochement desdites interfaces osseuses et une étape (iv) correspondant à l'étape (iii) ci-dessus.

Un type de matériel existant pour fixer ladite partie distale par rapport à ladite partie proximale comprend une plaque osseuse destinée à être fixée aux deux parties d'os au moyen de vis. Un autre type de matériel existant comprend une agrafe dont les branches latérales sont destinées à être insérées dans les deux parties d'os.

Ces matériels existants ont pour inconvénient de ne pas être toujours très faciles à implanter, compte tenu des dimensions réduites de la plaque osseuse ou de l'agrafe, et de ne pas exclure une fixation des deux parties d'os dans une position de correction relativement imprécise.

La présente invention a pour objectif de remédier à ces inconvénients essentiels.

Les documents US 2009/281577 et US 5,709,682 décrivent des plaques osseuses avec des tiges coudées solidaires d'elles, ces tiges coudées ayant des sections réduites permettant leur insertion dans les zones périphériques d'une partie d'os fracturée.

Ces matériels ne sont pas adaptés au traitement de petits os tels que les os de la main ou du pied.

La présente invention a aussi pour objectif de remédier à cet inconvénient.

Le matériel concerné comprend, de manière connue en soi,
- une plaque osseuse destinée à être fixée aux deux parties d'os, et
- une tige coudée à sensiblement quatre-vingt-dix degrés, dont une première branche est destinée à être engagée dans l'une des deux parties d'os.

Selon invention,
- ladite tige est une tige intramédullaire dont ladite première branche est destinée à être engagée dans le canal médullaire de ladite partie d'os, la deuxième branche de cette tige étant apte, lorsque ladite première branche est engagée dans le canal médullaire de ladite partie d'os, à être reliée à la plaque, et
- le matériel comprend des moyens de liaison de cette deuxième branche à la plaque.

Ainsi, après réalisation de ladite découpe transversale et pivotement de la partie distale de l'os par rapport à la partie proximale de celui-ci, ladite première branche de la tige intramédullaire, alors séparée de la plaque, est apte à engagée dans le canal médullaire de l'une ou l'autre des parties d'os (généralement dans la partie distale), ladite deuxième branche s'étendant entre les interfaces osseuses ; la plaque peut alors être raccordée à ladite première branche grâce auxdits moyens de liaison. Il en résulte que la plaque est maintenue par rapport aux parties d'os le temps que les vis de fixation de cette plaque à ces parties d'os soient mises en place, ce qui facilite grandement la mise en place de ces vis, particulièrement lorsqu'il s'agit de traiter des petits os tels que les os de la main ou du pied.

De préférence, le matériel comprend :
- au moins une pièce formant entretoise, ayant une forme en U, dont la partie centrale est destinée à être fixée à la plaque et dont les branches latérales ont une largeur déterminée, correspondant à la largeur d'un espace devant exister entre les interfaces osseuses selon la correction à réaliser, et
- des moyens de liaison à la plaque de cette pièce formant entretoise.

Cette pièce formant entretoise est destinée à être fixée à la plaque avec ses deux branches latérales faisant saillie de la face de la plaque destinée à être placée contre les parties d'os. Cette pièce permet, lorsque les interfaces osseuses sont en appui contre ses deux branches latérales, de placer les deux parties d'os dans une position précise l'une par rapport à l'autre, correspondant à la correction à réaliser, du fait que ces branches latérales ont une largeur correspondant à l'espace devant exister entre les interfaces osseuses selon la correction à réaliser. Cette pièce formant entretoise permet ainsi d'avoir l'assurance que les deux parties d'os sont dans la position de correction adéquate avant que l'immobilisation définitive des deux parties d'os soit réalisée au moyen des vis de fixation de la plaque aux parties d'os.

De préférence, le matériel comprend une pluralité de pièces formant entretoise, ayant des branches latérales de différentes largeurs.

La pièce ayant des branches de largeur adaptée à la correction à réaliser peut ainsi être choisie en fonction de l'espace devant exister entre les interfaces osseuses.

Ladite plaque pourrait présenter des ouvertures au travers desquelles lesdites branches latérales seraient aptes à être engagées ; de préférence, toutefois, la plaque présente, au niveau de sa partie destinée à se trouver en regard des interfaces osseuses, une largeur inférieure à celles de ses portions destinées à être fixées aux parties d'os, lesdites branches latérales de ladite pièce formant entretoise s'étendant, après liaison de cette pièce à la plaque, au-delà des bords de ladite partie de la plaque destinée à se trouver en regard des interfaces osseuses.

De préférence, ladite deuxième branche de la tige intramédullaire et la plaque comprennent des moyens de calage respectifs, propres à venir mutuellement en engagement lors de la liaison de cette deuxième branche à la plaque, de manière à réaliser un calage de la tige intramédullaire par rapport à la plaque selon l'axe de cette deuxième branche.

Un montage de la tige intramédullaire sur la plaque selon une position déterminée est ainsi obtenu.

Selon une forme de réalisation préférée de l'invention, dans ce cas, l'extrémité libre de ladite deuxième branche présente une portion prismatique, notamment hexagonale, et la plaque présente une ouverture de réception ajustée à cette portion prismatique.

Avantageusement, les moyens de liaison à la plaque de ladite deuxième branche de la tige intramédullaire constituent également les moyens de liaison à la plaque de la pièce formant entretoise.

Les liaisons à la plaque de la tige intramédullaire et de la pièce formant entretoise sont ainsi réalisées simultanément, par manoeuvre des mêmes moyens de liaison.

Selon une forme de réalisation préférée de l'invention, dans ce cas,
- ladite deuxième branche de la tige intramédullaire présente un alésage taraudé axial ;
- ladite branche centrale de la pièce formant entretoise comprend un trou la traversant de part en part, et
- le matériel comprend une vis propre à être engagée au travers de ladite branche centrale de la pièce formant entretoise et à être vissée dans l'alésage taraudé axial de ladite deuxième branche de la tige intramédullaire.

Avantageusement, la plaque comprend un évidement de réception au moins partielle de ladite branche centrale et de la base desdites branches latérales de la pièce formant entretoise.

Cet évidement permet d'assurer un positionnement précis de la pièce formant entretoise par rapport à la plaque et de réduire la hauteur de cette pièce formant entretoise par rapport à la plaque une fois le montage de la pièce formant entretoise réalisé.

Le matériel peut également comprendre une pièce de montage utilisable en lieu et place de ladite pièce formant entretoise lorsque ce matériel est utilisé pour opérer une correction consistant à rapprocher lesdites interfaces osseuses après coupe oblique au niveau de celles-ci (selon la deuxième technique exposée plus haut) ; cette pièce de montage a une forme correspondant à celle de la partie centrale de ladite pièce formant entretoise, et est dépourvue des branches latérales de celle-ci.

Le procédé de pose peut, dans ce cas, inclure une première étape consistant à percer la corticale proximale de l'os d'un trou permettant, même lorsque les interfaces osseuses sont rapprochées, de former un logement de réception de la tige intramédullaire.

L'invention sera bien comprise, et d'autres caractéristiques et avantages de celle-ci apparaîtront, en référence au dessin schématique annexé, représentant, à titre d'exemple non limitatif, une forme de réalisation préférée du matériel qu'elle concerne.
La figure 1 en est une vue en perspective, avant assemblage de différentes pièces qu'il comprend ;
la figure 2 en est une vue en perspective sous un autre angle, une fois cet assemblage réalisé ;
les figures 3 à 5, sont des vues d'une plaque osseuse que comprend ce matériel, respectivement de face, de côté et en bout ;
la figure 6 est une vue de dessus d'une pièce de montage qu'il comprend ;
la figure 7 est une vue de côté de cette même pièce de montage ;
la figure 8 est une vue des os d'un pied, le premier métatarsien ayant une position défectueuse qui nécessite la réalisation d'une chirurgie correctrice de cette position ;
la figure 9 est une vue similaire à la figure 8, après mise en oeuvre sur le premier métatarsien de premières étapes de cette chirurgie correctrice, et
les figures 10 à 12 sont des vues partielles, similaires à la figure 9, à échelle agrandie, du premier métatarsien au cours de trois étapes successives de mise en oeuvre de cette chirurgie correctrice.

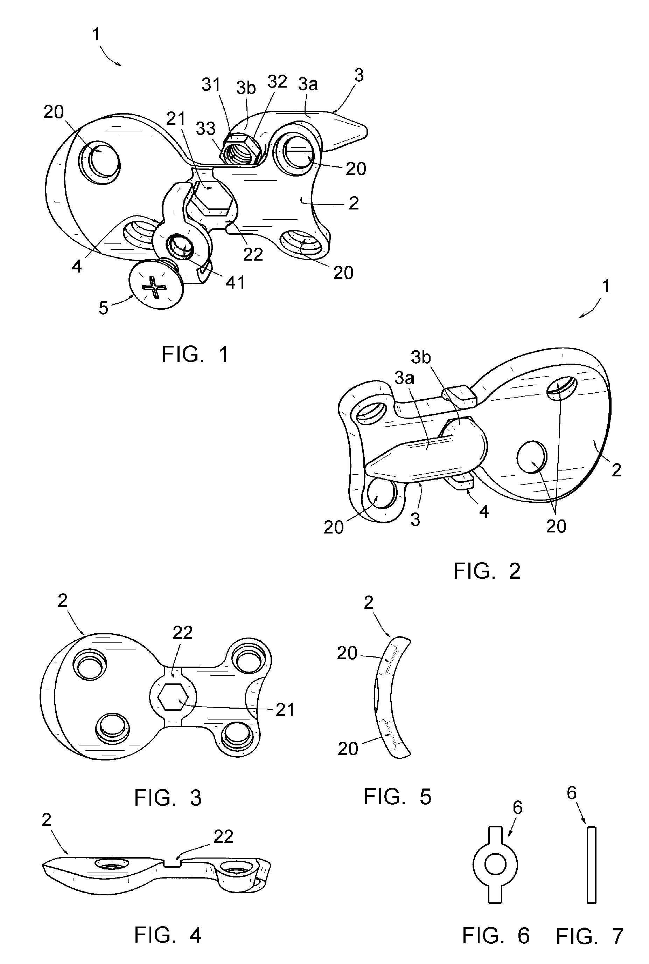

Les figures 1 et 2 représentent un matériel 1 de fixation de deux parties d'un os l'une à l'autre, permettant en particulier d'opérer une chirurgie correctrice sur le premier métatarsien afin de traiter la pathologie connue sous le nom d'hallux valgus. La figure 8 montre un premier métatarsien 100 dont la position défectueuse peut, au moyen de ce matériel 1, être corrigée vers la position de correction montrée sur les figures 9 à 12.

Le matériel 1 comprend une plaque osseuse 2, une tige intramédullaire 3, plusieurs pièces 4 formant entretoise (une seule est représentée sur ces figures) et une vis d'assemblage 5. Il comprend également la pièce de montage 6 montrée sur les figures 6 et 7.

La plaque osseuse 2 est destinée à être fixée aux deux parties d'os et, comme cela est visible notamment sur la figure 5, présente à cet effet une courbure transversale adaptée à la courbure des parties d'os à maintenir ainsi que quatre trous 20 au travers desquels sont destinées à être engagées des vis d'ancrage (non représentées). Chaque trou 20 comprend un lamage permettant d'effacer au moins partiellement la tête de la vis dans l'épaisseur de la plaque 2.

Au niveau de sa partie centrale, la plaque 2 présente une largeur moindre et comprend une ouverture traversante 21 de forme hexagonale. Cette ouverture 21 débouche dans la zone centrale circulaire d'un évidement 22 aménagé sur la face convexe de la plaque 2, cette zone centrale étant raccordée à chacun des bords longitudinaux de la plaque 2 par des parties latérales d'évidement s'étendant de manière radiale rapport à la zone centrale en étant diamétralement opposées.

La tige intramédullaire 3 est coudée à sensiblement quatre-vingt-dix degrés, et présente une première branche 3a destinée à être engagée dans le canal médullaire de l'une des deux parties d'os et une deuxième branche 3b apte, lorsque ladite première branche 3a est engagée dans le canal médullaire de ladite partie d'os, à être reliée à la plaque 2.

La première branche 3a présente une extrémité libre conique favorisant son insertion dans ce canal médullaire de ladite partie d'os.

La deuxième branche 3b comprend, au niveau de son extrémité libre, une portion hexagonale 31 propre à être engagée de manière ajustée dans l'ouverture traversante hexagonale 21 de la plaque 2, jusqu'à prise d'appui de la face d'extrémité circulaire 32 de la tige 3 située à proximité de la base de cette portion 31 contre la face concave de la plaque 2.

Cette deuxième branche 3b est par ailleurs percée d'un alésage taraudé axial 33 propre à recevoir le corps fileté de la vis 5 comme décrit plus loin.

Chaque pièce 4 formant entretoise a une forme en U, présentant une branche centrale et deux branches latérales formant corps avec cette branche centrale, s'étendant sensiblement perpendiculairement à celle-ci.

La branche centrale présente une portion médiane de forme circulaire, percée d'un trou traversant 41 permettant le passage au travers de lui du corps fileté de la vis 5, et deux portions latérales diamétralement opposées, se raccordant auxdites branches latérales de la pièce 4.

Ainsi que cela se comprend en référence aux figures 1 et 2, la portion hexagonale 31 est destinée à être engagée de manière ajustée dans l'ouverture 21 de la plaque 2, la branche centrale de la pièce 4 est destinée à être engagée dans l'évidement 22, avec sa portion centrale circulaire prenant place dans la partie centrale de cet évidement 22 et ses portions latérales prenant place dans lesdites parties latérales de cet évidement 22, puis le corps fileté de la vis 5 est destiné à être engagé dans le trou 41 et dans l'alésage taraudé 33 de ladite deuxième branche 3b, pour constituer l'ensemble montré sur la figure 2. Dans cet ensemble, la tige 3 et la pièce 4 sont reliées à la plaque 2, ladite première branche 3a s'étendant sensiblement parallèlement à la génératrice médiane de la plaque 2, et les branches latérales de la pièce 4 faisant saillie de la face concave de la plaque 2, sensiblement dans le même plan transversal que celui contenant l'axe de la deuxième branche 3b.

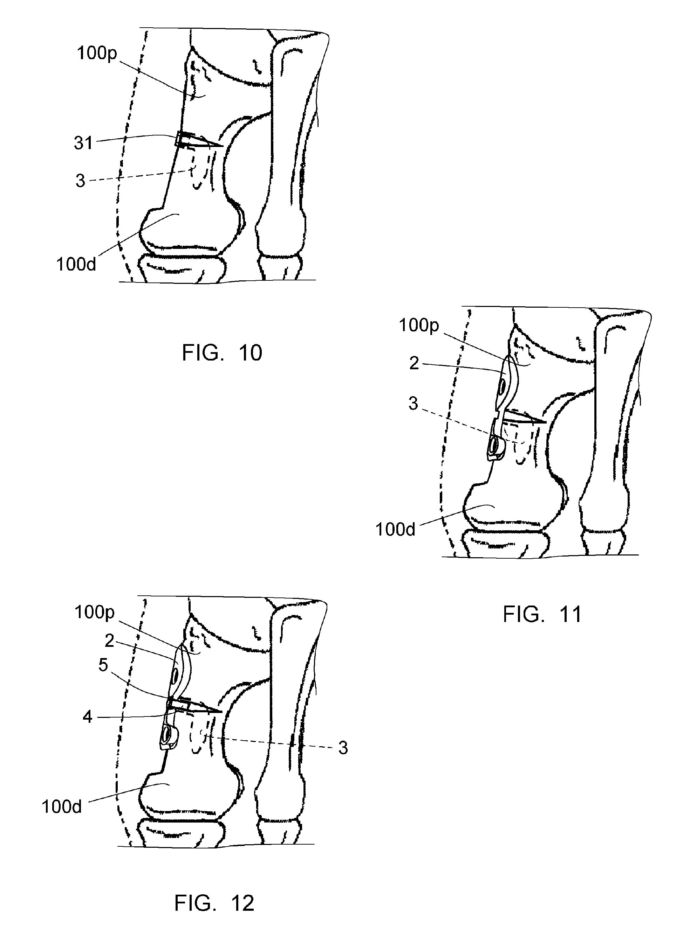

En pratique, pour traiter la position défectueuse du premier métatarsien 100 montré sur la figure 8, il est procédé comme suit :
- une résection de la face interne du premier métatarsien 100 est opérée afin d'aménager un emplacement 101 de réception ultérieure de la plaque 2 ;
- une fente transversale est aménagée dans le premier métatarsien 100, à partir de la face interne de celui-ci, sur pratiquement la totalité de sa section, c'est-à-dire en laissant subsister une portion réduite 102 de jonction entre les deux parties d'os 100p, 100d individualisées par la fente ; cette portion réduite 102 est suffisante pour maintenir une liaison des parties d'os mais est propre à permettre un pivotement de la partie distale 100d de l'os par rapport à la partie proximale 100p de celui-ci ;
- un pivotement de la partie distale 100d par rapport à la partie proximale 100p est opéré dans le sens de l'éloignement des interfaces osseuses de ces parties d'os, comme cela est visible sur la figure 9, jusqu'à obtention de la position corrigée recherchée ; un espace 103 en forme de V est ainsi généré au niveau des interfaces osseuses aménagées par ladite fente ;
- la première branche 3a de la tige 3 est ensuite engagée dans le canal médullaire de la partie d'os distale 100d à partir de l'espace 103, la deuxième branche 3b de cette tige prenant place dans cet espace 103, avec sa portion hexagonale 31 dépassant légèrement de la corticale osseuse réséquée (cf. figure 10) ;
- la plaque 2 est ensuite placé contre les parties d'os de manière à engager ladite portion hexagonale 31 dans l'ouverture 21 (cf. figure 11) ;
- la pièce 4 formant entretoise adaptée à la correction à réaliser est ensuite mise en place sur la plaque 2 puis la vis 5 est vissée dans l'alésage 33 au travers de la pièce 4 et de la plaque 2 (cf. figure 12), réalisant ainsi un montage provisoire de l'ensemble.

Des vis sont ensuite mises en place au travers des trous 20 de la plaque 2, selon un processus facilité par le maintien en position de la plaque 2 contre l'os 100.

Si, selon la position défectueuse du premier métatarsien 100, une correction doit être opérée dans le sens opposé à celui mentionné ci-dessus, le procédé comprend les deux premières étapes ci-dessus (résection de la face interne du premier métatarsien 100 et aménagement de la fente transversale), puis une étape consistant à opérer une coupe osseuse oblique au niveau de chaque interface osseuse, afin d'aménager un espace vide entre ces interfaces, puis une étape consistant à opérer un pivotement de la partie distale par rapport à la partie proximale dans le sens du rapprochement desdites interfaces osseuses et une étape de fixation de la plaque 2 correspondant à l'étape ci-dessus.

Dans ce cas, la pièce de montage 6 est utilisée en lieu et place de la pièce 4 formant entretoise. Cette pièce de montage 6 a une forme correspondant à celle de la partie centrale de la pièce 4 formant entretoise, et est dépourvue des branches latérales de celle-ci ; elle prend place dans l'évidement 22 et permet de former une surface d'appui à la tête de la vis 5.

Le procédé de pose peut inclure une toute première étape consistant à percer la corticale proximale de l'os d'un trou permettant, même lorsque les interfaces osseuses sont rapprochées, de former un logement de réception de la tige intramédullaire 2.

Ainsi que cela apparaît de ce qui précède, l'invention fournit un matériel 1 de fixation de deux parties d'un os l'une à l'autre, notamment pour le traitement d'un hallux valgus, présentant les avantages déterminants être facile à implanter nonobstant les dimensions réduites de la plaque 2, et d'exclure tout risque de fixation des deux parties d'os 100p, 100d dans une position de correction imprécise.

Le matériel 1 comprend plusieurs pièces 4 dont les branches latérales présentent différentes largeurs, adaptées à différentes largeurs d'espaces 103 devant exister entre les interfaces des deux parties d'os selon la correction à réaliser. Par exemple, le matériel 1 peut comprendre une pièce 4 dont les branches latérales ont une largeur adaptée à une correction de 10° (angle entre les axes médians des deux parties d'os après correction), une autre pièce 4, dont les branches latérales ont une largeur adaptée à une correction de 15° et une autre pièce 4 dont les branches latérales ont une largeur adaptée à une correction de 20°.

L'invention a été décrite ci-dessus en référence à une forme de réalisation fournie à titre d'exemple. Il va de soi qu'elle n'est pas limitée à cette forme de réalisation mais qu'elle s'étend à toutes les autres formes de réalisation couvertes par les revendications ci-annexées. Notamment, la portion hexagonale 31 et l'ouverture hexagonale 21 peuvent être remplacées par toute autre forme de moyens de calage de la tige 3 par rapport à la plaque 2.

## Revendications

1. Matériel (1) de fixation de deux parties d'un os l'une à l'autre, comprenant :
- une plaque osseuse (2) destinée à être fixée aux deux parties d'os, et
- une tige (3) coudée à sensiblement quatre-vingt-dix degrés, dont une première branche (3a) est destinée à être engagée dans l'une des deux parties d'os ;
**caractérisé :**
- **en ce que** ladite tige (3) est une tige intramédullaire dont ladite première branche (3a) est destinée à être engagée dans le canal médullaire de ladite partie d'os, la deuxième branche (3b) de cette tige étant apte, lorsque ladite première branche (3a) est engagée dans le canal médullaire de ladite partie d'os, à être reliée à la plaque (2), et
- **en ce que** le matériel comprend des moyens (5, 21, 33) de liaison de cette deuxième branche (3b) à la plaque (2).

2. Matériel (1) selon la revendication 1, **caractérisé en ce qu'**il comprend :
- au moins une pièce (4) formant entretoise, ayant une forme en U, dont la partie centrale est destinée à être fixée à la plaque (2) et dont les branches latérales ont une largeur déterminée, correspondant à la largeur d'un espace devant exister entre les interfaces osseuses selon la correction à réaliser, et
- des moyens (5, 21, 33) de liaison à la plaque (2) de cette pièce (4) formant entretoise.

3. Matériel (1) selon la revendication 2, **caractérisé en ce qu'**il comprend une pluralité de pièces (4) formant entretoise, ayant des branches latérales de différentes largeurs.

4. Matériel (1) selon l'une des revendications 2 ou 3, **caractérisé en ce que** la plaque (2) présente, au niveau de sa partie destinée à se trouver en regard des interfaces osseuses, une largeur inférieure à celles de ses portions destinées à être fixées aux parties d'os, lesdites branches latérales de ladite pièce (4) formant entretoise s'étendant, après liaison de cette pièce (4) à la plaque (2), au delà des bords de ladite partie de la plaque (2) destinée à se trouver en regard des interfaces osseuses.

5. Matériel (1) selon l'une des revendications 1 à 4, **caractérisé en ce que** ladite deuxième branche (3b) de la tige intramédullaire et la plaque (2) comprennent des moyens de calage respectifs (21, 31), propres à venir mutuellement en engagement lors de la liaison de cette deuxième branche (3b) à la plaque (2), de manière à réaliser un calage de la tige intramédullaire (3) par rapport à la plaque (2) selon l'axe de cette deuxième branche (3b).

6. Matériel (1) selon la revendication 5, **caractérisé en ce que** l'extrémité libre de ladite deuxième branche (3b) présente une portion prismatique (31), notamment hexagonale, et **en ce que** la plaque (2) présente une ouverture de réception (21) ajustée à cette portion prismatique (31).

7. Matériel (1) selon l'une des revendications 2 à 6, **caractérisé en ce que** les moyens (5, 21, 33) de liaison à la plaque (2) de ladite deuxième branche (3b) de la tige intramédullaire (3) constituent également les moyens de liaison à la plaque (2) de la pièce (4) formant entretoise.

8. Matériel (1) selon la revendication 7, **caractérisé en ce que** :
- ladite deuxième branche (3b) de la tige intramédullaire présente un alésage taraudé (33) axial ;
- ladite branche centrale de la pièce (4) formant entretoise comprend un trou (41) la traversant de part en part, et
- le matériel (1) comprend une vis (5) propre à être engagée au travers de ladite branche centrale de la pièce (4) formant entretoise et à être vissée dans l'alésage taraudé axial (33) de ladite deuxième branche (3b) de la tige intramédullaire (3).

9. Matériel (1) selon l'une des revendications 2 à 8, **caractérisé en ce que** la plaque (2) comprend un évidement (22) de réception au moins partielle de ladite branche centrale et de la base desdites branches latérales de la pièce (4) formant entretoise.

10. Matériel (1) selon l'une des revendications 2 à 9, **caractérisé en ce qu'**il comprend une pièce de montage (6) ayant une forme qui correspond à celle de la partie centrale de ladite pièce (4) formant entretoise, et qui est dépourvue des branches latérales de celle-ci.
